# EUROPEAN PATENT SPECIFICATION

(11) **EP 2 440 247 B1**
(45) Date of publication and mention of the grant of the patent: **08.03.2017**
(21) Application number: 09788193.2
(22) Date of filing: 12.06.2009
(51) Int. Cl.: A61K 41/00, A61K 47/69, A61K 49/00, A61K 49/18, A61P 35/00, B82Y 5/00

(54) **TARGETED NANO-PHOTOMEDICINES FOR PHOTODYNAMIC THERAPY OF CANCER**
ZIELGERICHTETE NANOPHOTOMEDIKAMENTE ZUR PHOTODYNAMISCHEN BEHANDLUNG VON KREBS
NANO-PHOTOMÉDICAMENTS CIBLÉS DESTINÉS AU TRAITEMENT PHOTODYNAMIQUE DU CANCER

(43) Date of publication of application: 18.04.2012
(73) Proprietor: Erasmus University Medical Center Rotterdam, 3015 GE Rotterdam (NL); Amrita Vishwa Vidyapeetham University, Kerala PO-682026 (IN)
(72) Inventor: KOYAKUTTY, Manzoor, Kerala PO-682026 (IN); ROBINSON, Dominic James, NL-3015 GE Rotterdam (NL); STERENBORG, Henricus Johannes Cornelius Maria, NL-3015 GE Rotterdam (NL); KASCAKOVA, Slavka, NL-3015 GE Rotterdam (NL); NAIR, Shantikumar, PO-682026 (IN)
(74) Representative: V.O.
(86) International application number: PCT/NL2009/050337
(87) International publication number: WO 2010/143942

(56) References cited:
- EP-A1- 1 097 935
- WO-A1-95/27481
- WO-A1-2008/130181
- WO-A1-2008/133598
- WO-A1-2009/077908
- WO-A2-2006/060797
- WO-A2-2007/136413
- DATABASE CA [Online] CHEMICAL ABSTRACTS SERVICE, COLUMBUS, OHIO, US; 2007, MCCARTHY, JASON R. ET AL: "Multimodal nanoagent for the diagnosis and treatment of atherosclerosis" XP002586815 retrieved from STN Database accession no. 2007:883487 & MCCARTHY, JASON R. ET AL: "Multimodal nanoagent for the diagnosis and treatment of atherosclerosis" ABSTRACTS OF PAPERS, 234TH ACS NATIONAL MEETING, BOSTON, MA, USA, MEDI-122, PUBLISHER: AMERICAN CHEMICAL SOCIETY, WASHINGTON, D. C., 19 August 2007 (2007-08-19), - 23 August 2007 (2007-08-23)
- GUO YANLING ET AL: "Amphiphilic block copolymeric micelles as chlorin e6 carriers" LIFE SCIENCE JOURNAL-ACTA ZHENGZHOU UNIVERSITY OVERSEAS EDITION, vol. 5, no. 1, 2008, pages 1-5, XP002586816 ISSN: 1097-8135
- HAMBLIN MICHAEL R ET AL: "Pegylation of a chlorine6 polymer conjugate increases tumor targeting of photosensitizer" CANCER RESEARCH, vol. 61, no. 19, 1 October 2001 (2001-10-01), pages 7155-7162, XP002586817 ISSN: 0008-5472
- BACHOR R ET AL: "Photosensitized destruction of human bladder carcinoma cells treated with chlorin e6-conjugated microspheres" PROCEEDINGS OF THE NATIONAL ACADEMY OF SCIENCES OF THE UNITED STATES OF AMERICA, vol. 88, no. 4, 15 February 1991 (1991-02-15), pages 1580-1584, XP002586818 ISSN: 0027-8424
- BACHOR R ET AL: "FREE AND CONJUGATED CHLORIN E-6 IN THE PHOTODYNAMIC THERAPY OF HUMAN BLADDER CARCINOMA CELLS" JOURNAL OF UROLOGY, vol. 146, no. 6, 1991, pages 1654-1658, XP8122904 ISSN: 0022-5347
- DEKKER, L., ET AL: "Lethal photosensitisation of Staphylococcus aureus using a tin chlorin e6-gold nanoparticle" PHOTODIAGNOSIS AND PHOTODYNAMIC THERAPY: EPPM - 1 ABSTRACTS, vol. 5, no. 1, 1 March 2008 (2008-03-01), pages 62-96, XP022586448 ISSN: 1572-1000 [retrieved on 2008-03-04]
- TOKUOKA, YOSHIKAZU ET AL: "Anticancer effect of dye-sensitized TiO2 nanocrystals by polychromatic visible light irradiation" CHEMISTRY LETTERS, vol. 35, no. 5, 2006, pages 496-497, XP8123019 ISSN: 0366-7022
- SCHMIDT-ERFURTH U ET AL: "PHOTODYNAMIC THERAPY IN OCULAR VASCULAR DISEASE" LASER PHYSICS, vol. 8, no. 1, 1 January 1998 (1998-01-01) , pages 191-198, XP000856256 ISSN: 1054-660X
- PEGAZ B ET AL: "Encapsulation of porphyrins and chlorins in biodegradable nanoparticles: The effect of dye lipophilicity on the extravasation and the photothrombic activity. A comparative study" JOURNAL OF PHOTOCHEMISTRY AND PHOTOBIOLOGY B: BIOLOGY, vol. 80, no. 1, 1 July 2005 (2005-07-01), pages 19-27, XP025302168 ISSN: 1011-1344 [retrieved on 2005-07-01]
- BACHOR R ET AL: "MECHANISM OF PHOTOSENSITIZATION BY MICROSPHERE-BOUND CHLORIN E6 IN HUMAN BLADDER CARCINOMA CELLS" CANCER RESEARCH, vol. 51, no. 16, 15 August 1991 (1991-08-15), pages 4410-4414, XP008055015 ISSN: 0008-5472
- ZHU ZHI ET AL: "Regulation of singlet oxygen generation using single-walled carbon nanotubes." JOURNAL OF THE AMERICAN CHEMICAL SOCIETY, vol. 130, no. 33, 20 August 2008 (2008-08-20), pages 10856-10857, XP002586821 ISSN: 1520-5126
- SHEEN VOLNEY L ET AL: "Apoptotic mechanisms in targeted neuronal cell death by chromophore-activated photolysis" EXPERIMENTAL NEUROLOGY, vol. 130, no. 1, 1994, pages 67-81, XP002586822 ISSN: 0014-4886

## Description

### FIELD OF THE INVENTION

The present invention relates to cancer therapy and therapeutic formulations for use in the treatment of cancer. In particular, the present invention relates to nanomedicines for use in photodynamic therapy of cancer, as well as methods for preparing said nanomedicines.

### BACKGROUND OF THE INVENTION

Photodynamic therapy (PDT) is an emerging treatment modality for the treatment of many types of cancers and various non-malignant conditions. In PDT, light activation of a photosensitizer drug creates reactive oxygen species (ROS), such as singlet oxygen (¹O₂), free radicals or peroxides that can oxidatively destroy cellular compartments including plasma, mitochondria, lysosomal, and nuclear membranes, resulting in irreversible damage of tumor cells. Under appropriate conditions, photodynamic therapy offers the advantage of an effective and selective method of destroying diseased tissues without damaging adjacent healthy ones. However, despite PDT's advantages over current treatments (e.g. surgery, radiation therapy, and chemotherapy), its general clinical acceptance as a mainstream cancer therapy tool is still very low. This is because of some critical limitations of current PDT technique such as pro-longed photosensitivity of the body due to nonspecific biodistribution of the photosensitive drug, low photo absorption of the drug at better tissue penetrating regions of light spectrum, hydrophobicity of PS drugs leading to uncontrolled aggregation in circulation and difficulties in administration, fast photobleaching of hydrophilic drugs, non-specific drug localization leading to lack of optimum concentration of drug at target sites.

Given the lack of effective targeting of traditional approaches to PDT the state of the art has and continues to develop conjugates for targeted photodynamic therapy (the conjugate combines the photosensitizer with a targeting ligand e.g. monoclonal antibodies, peptides, folic acid, etc). It is important to note that these approaches are closely related to the development of targeted optical diagnostic conjugates which incorporate small fluorescent molecules conjugated to the same targeting ligands that are used in targeted photodynamic therapy. However state of the art targeted PDT has a number of significant challenges. 1) Most effective photosensitizes are hydrophobic in nature with inherently poor water solubility and have a high affinity for lipidic environments. This has two consequences: First when photosensitizer conjugates are injected at physiological conditions they form aggregates that bind to plasma proteins and are removed from the host by the endoreticular system. This limits the effective concentration of conjugate that can be achieved in any target tissue. Second when the photosensitizer conjugates interact with the target cells their high lipophilicity promotes non-specific cellular uptake. This process competes with active receptor targeting and lead to conjugate accumulation in normal cells that do not express the target receptor. 2) The fact that a single photosensitizer molecule is attached to a single targeting ligand means that there can be a limit to the amount of photosensitizer that can be incorporated in to cells with a finite number of receptors. While efforts have been made to attach multiple photosensitizer molecules (or their pre-cursors) to a single targeting ligand this is remains a significant problem. Also one important property of free photosensitizers is that are themselves destroyed by the generation of reactive oxygen species, a similar effect occurs in photosensitizer-ligand conjugates. This effect limits the total dose of reactive oxygen that can be delivered to tissue. Achieving a high concentration of photosensitizer per receptor is therefore critical.

McCarthy et al, (Database Chemical abstracts: Multimodal nanoagent for the diagnosis and treatment of atherosclerosis) disclose cross-linked iron-oxide nanoparticles which are conjugated to AlexaFluor 750. The photosensitizer (AlexaFluor 750) is coupled to the nanoparticle after assembly of the nanoparticle.

EP 1 097 935 discloses gold complexes of chlorin e6 (a photosensitizer).

WO2007/136413 discloses Fluorescent metal oxide nanoparticles that may be linked to biomolecules and other molecules such as photosensitizers. First nanoparticles from metal oxides are made before fluorochromes are linked.

WO2006/060797 is directed to MRI guided photodynamic therapy for cancer. It discloses MRI contrast agent labelled polymer photosensitizer conjugates.

Guo et al. (Amphiphilic block copolymer micelles as chlorin e6 carriers, Life Science Journal, Acta, Zhengzhou University Overseas edition, vol 5, no 1, 2008, pages 1-5) disclose block copolymeric micelles as chlorin e6 carriers. Guo first makes first micelles, after which the chlorin e6 is introduced. The chlorin e6 is free in the micelle.

Hamblin et al: (Pegylation of a chlorin e6 polymer conjugate increases tumor targeting of photosensitizer, Cancer Research vol 61, no 19, (2001) page 7155-7162) discloses pegylation of chlorin e6 conjugated polymers.

Bachor et al. (Photosensitized destruction of human bladder carcinoma cells treated with chlorin e6-conjugated microspheres, Proceedings of the National Academy of Sciences of the United States of America, vol 88, no 4 (1991), pages 1580-1584) teach the use of chlorin e6 conjugated microspheres.

Bachor et al: (Free and conjugated chlorin e6 in the photodynamic therapy of human bladder carcinoma cells, Journal of Urology vol 146, no 6 (1991) pages 1654-1658) show a study between free and conjugated chlorin e6 to microspheres.

Dekker et al. (Lethal photsensitisation of Staphylococcocus aureus using a tin chlorin e6-gold nanoparticle, Photodiagnosis and photodynamic therapy: EPPM-1 Abstracts vol 5, no 1, (2008) pages 62-96) disclose chlorin e6 gold nanoparticles wherein chlorin e6 is coupled to an already made gold particle through a glutathione linker.

Tokuoka et al. (Anticancer effect of dye-sensitized TiO2 nanocrystals by polychromatic visible light irradiation, Chemistry Letters vol. 35, no. 5, 2006, pages 496-497) show anticancer effects of dye sensitized TiO2 nanocrystals.

WO2008/130181 is directed to photosensitizer conjugated polymers for photodynamic therapy.

Schmidt-Erfurth et al. (Photodynamic therapy in ocular vascular disease, Laser Physics, vol 8, no 1 (1998) pages 191-198) disclose LDL conjugated to photosensitizers for use in photodynamic therapy.

WO95/27481 discloses liquid delivery compositions for sustained release systems which may comprise nanoparticles. It discloses that the active agent may be distributed throughout the nanosphere.

Pegaz et al (Encapsulation of porphyrins and chlorins in biodegradable nanoparticles: The effect of dye lipophilicity on the extravasation and the photothrombic activity. A comparative study. Journal of Photochemistry and Photobiology B: Biology, vol 80, no 1, (2005) pages 19-27) describe the encapsulation of porphyrins and chlorins in biodegradable nanoparticles for photodynamic therapy.

Bachor et al: (Mechanism of photosenzitation by mircrosphere-bound chlorin e6 in human bladder carcinoma cells, Cancer Research vol 51, no 15; (1991), pages 4410-4414) disclose microsphere bound chlorin e6 in bladder carcinoma cells. The chlorin e6 is conjugated to already made polystyrene microspheres.

Zhu Zhi et al. (Regulation of singlet oxygen generation using singlewalled carbon nanotubes, Journal of the American Chemical Society vol 130, no 33 (2008), pages 10856-10857) describe the use of nanotubes for singlet oxygen generation. Ready made nanotubes are coupled to a photosensitizer chlorin e6) via an aptamer.

Sheen et al. (Apoptotic mechanisms in targeted neuronal cell death by chromophore-activated photolysis, Experimental Neurology, vol 130, no 1 (1994) pages 67-81) disclose nanospheres carrying chlorin e6 that are used in neuroblastoma cells. The chlorin e6 was conjugated to already made latex nanospheres.

WO2008/133598 discloses the formation of cross-linked glutathione on nanostructures. A cross-linked glutathione layer was coated on a surface of a metal based nanostructure.

WO2009/077908 is directed to colloidal particle comprising multivalent cyclic anions. The particles are formed through electrostatic forces.

### SUMMARY OF THE INVENTION

In a first aspect, the present invention provides a method for the production of a photosensitizer-containing nanoparticle suitable for use in photodynamic therapy comprising:
- providing a nanoparticle precursor molecule;
- coupling a photosensitizer to said nanoparticle precursor molecule to provide a photosensitizer-conjugated nanoparticle precursor, and
- forming a nanoparticle from said photosensitizer-conjugated nanoparticle precursor by solution-precipitation or self assembly of said nanoparticles precursor.

In a highly preferred embodiment, the present invention provides a method for the production of a photosensitizer-containing nanoparticle suitable for use in molecular imaging assisted targeted photodynamic therapy comprising:
- providing a nanoparticle precursor molecule;
- coupling a photosensitizer to said nanoparticle precursor molecule to provide a photosensitizer-conjugated nanoparticle precursor,
- incorporating a magnetic and/or optical contrast agent to the photosensitizer-nanoparticle precursor conjugate, and
- forming a nanoparticle from said photosensitizer-nanoparticle precursor mixture containing magnetic and/or optical contrast agent by solution-precipitation or molecular self assembly.

In a preferred embodiment of said method, said nanoparticles is formed from a material selected from the group consisting of metal sulphate, metal phosphate, metal oxide, chitosan, carboxymethyl chitosan (CMC), polyvinyl alcohol (PVA), polystyrene (PS) polyvinylpyrrolidone (PVP), polylactic acid (PLA), polyethylenimine (PEI), poly(lactic-co-glycolic acid) (PLGA), polycaprolactone (PCL), polyethylene glycol (PEG), and combinations thereof.

In another preferred embodiment of said method, said metal oxide is silica, said precursor molecule is an orthosilica and said nanoparticle is formed by a sol-gel process for the formation of silicate powders by hydrolysis and condensation under conditions of basic pH and under sonication to form colloidal silica nanoparticles.

In yet another preferred embodiment of said method, said photosensitizer is selected from chlorin e₆ (Ce₆), meso-tetra(3-hydroxyphenyl)chlorin (m-THPC), benzoporphyrin derivative monoacid ring A (BPD or verteporfin), photofrin, temoporfin (Foscan®), Rose bengal, metal phthalocyanine and combinations thereof.

In another aspect, the present invention provides a photosensitizer-containing nanoparticle obtainable by a method according to the present invention as described above.

In another aspect, the present invention provides a photosensitizer-containing nanoparticle, comprising a photosensitizer covalently bonded throughout at least a part of said nanoparticle to the nanoparticle matrix material and incorporated therein as a mixture of monomeric and aggregated molecules, wherein the ratio of Q band absorption to Soret band absorption of said nanoparticles has a value of at least 0.3.

In a preferred embodiment of aspects of the invention, said nanoparticle is formed from a material selected from the group consisting of metal sulphate, metal phosphate, metal oxide, chitosan, polyvinylpyrrolidone (PVP), polylactic acid (PLA), polyethylenimine (PEI), poly(lactic-co-glycolic acid) (PLGA), and combinations thereof.

In a highly preferred embodiment of the invention said nanoparticle is formed from a metal oxide, and preferably said metal oxide is silica.

In yet another preferred embodiment of a nanoparticle according to the present invention, said photosensitizer is selected from chlorin e₆ (Ce₆), meso-tetra(3-hydroxyphenyl)chlorin (m-THPC), benzoporphyrin derivative monoacid ring A (BPD or verteporfin), photofrin, temoporfin (Foscan®), Rose bengal, metal phthalocyanine and combinations thereof.

In yet another preferred embodiment of a nanoparticle according to the present invention, said nanoparticle is doped with an optical contrast agent and/or a magnetic contrast functionality.

In yet another preferred embodiment, the optical contrast agent is luminescent quantum dots of ZnS doped with Mn, Cu-Al or Cu-halogen.

In yet another preferred embodiment, the magnetic contrast functionality is provided by doping the nanophotomedicine with Gd³⁺, Fe³⁺ or Mn²⁺.

In still another preferred embodiment, said nanoparticle comprises a cancer-targeting ligand connected to the outermost surface through covalent linkage. Preferably the cancer-targeting ligand is octreotide.

In another aspect, the present invention provides an injectable composition or composition for oral administration comprising the nanoparticles according to the present invention as described above, together with a pharmaceutically acceptable carrier.

In another aspect, the present invention provides a method of killing cancer cells by PDT treatment, comprising contacting said cancer cells with a nanoparticle according to the present invention as described above and irradiating said nanoparticles with a therapeutically effective amount of light so as to evoke singlet oxygen emission from said nanoparticles.

In another aspect, the present invention provides a method of killing cancer cells by image assisted PDT treatment, comprising contacting said cancer cells with a nanoparticle according to the present invention as described above and irradiating said nanoparticles with a therapeutically effective amount of light so as to evoke singlet oxygen emission from said nanoparticles, wherein the nanoparticle is doped with an optical contrast agent and/or a magnetic contrast agent and wherein the direction of said irradiation is guided by imaging techniques that use the optical or magnetic contrast agent as markers to indicate the location, size and spread of the cancer cells.

### DESCRIPTION OF THE DRAWINGS

Figure 1 shows the results of the experiment described in Example 1: Transmission electron micrograph of silica based nanophotomedicine of size 90-100nm.
Figure 2 shows the results of the experiment described in Examples 1-3: Fluorescence excitation spectra nanophotomedicine based on Chlorin e6@silica showing systematic increase in the absorption of NPM-1, NPM-2 and NPM-3 at 654nm due to specific processing condition, compared to that of free photomedicine.
Figure 3 shows the results of the experiment described in Example 4: Photoluminescence excitation spectra of nanophotomedicine based on mTHPC@silica showing complete modification of excitation spectra leading to ∼6 fold enhancement in red absorption band compared to that of free-mTHPC.
Figure 4 shows the results of the experiment described in Example 5: Photodegradation properties of nanophotomedicine is compared with free-chorine e6 having nearly same initial fluorescence intensity. Free - Ce6 showed very fast photobleaching property by singlet oxygen produced during photosensitization whereas NPM showed completely different non-linear bleaching characteristics resulting extended photostability of the drug even after 10J of irradiation.
Figure 5 shows the results of the experiment described in Example 6: Confocal microscopic image of the fast photobleaching of free-chlorin e6 within the cancer cells under laser irradiation (405nm,30Sec, left panel), whereas in nanophotomed treated cells photoactivity of the drug is still shown even after prolonged irradiation (360sec, right panel).
Figure 6 shows the results of the experiment described in Example 7: a) X-Ray diffraction, b) photoluminescence (PL) spectra and c) digital photograph showing fluorescence emission from ZnS:Mn QD doped nanophotomedicines.
Figure 7 shows the results of the experiment described in Example 10: Vibrating sample magnetometer data showing paramagnetic property of Gd³⁺ doped nanophotomedicine, suitable for MRI imaging as against diamagnetic property of free-photomedicine (Ce₆).
Figure 8 shows the results of the experiment described in Example 10: MRI phantom imaging of nanophotomedicines (NPM) of different concentration with reference to water and free-Ce₆.
Figure 9 shows the results of the experiment described in Example 11: Confocal microscopic image showing efficient intracellular uptake of peptide conjugated nanophotomedicine by sst2 receptor +ve cancer cells (K562).
Figure 10 shows the results of the experiment described in Example 11: Photodynamic therapy data of cancer cells showing enhanced cell death (low cell viability) in nanophotomedicine treated K562 cells compared to free chlorin e6 of same concentration and control nanoparticles. Fig 10 shows depending on the absorbance at 654nm, the PDT effect of NPMs are better

### DETAILED DESCRIPTION OF THE INVENTION

### Definitions

The term "nanoparticle" as used herein, refers to a crystallite or primary particle measuring about 20-500 nm, preferably 50-200nm, most preferably around 100 nm in size. The nanoparticle may be an organic or inorganic nanoparticle including a polymeric nanoparticle. The particles may be produced in the form of dry powders or liquid dispersions. Generally, nanoparticles in the form of higher value-added products require further processing to provide slurries, films or devices. In the present invention the application as device is envisioned. The nanoparticles may be solid or porous and may comprise an inner core surrounded by one or more continuous or semi-continuous shells or may comprise a single monolithic particle. Both, the core and shell(s) may be organic, inorganic or polymeric. Suitable nanoparticulate materials for the manufacture of the nanoparticle are simple metal oxides, such as silica (SiO₂), titania (TiO₂), alumina (Al₂O₃), iron oxide (Fe₃O₄, Fe₂O₃), zinc oxide (ZnO), ceria (CeO₂) and zirconia (ZrO₂). Also suitable are mixed oxides, such indium-tin oxide (In₂O₃-SnO₂ or ITO) and antimony-tin oxide (ATO), silicates (aluminum and zirconium silicates) and titanates (barium titanate (BaTiO₃)). Other types of nanoparticles, including various complex oxides, semiconductors, nonoxide ceramics (e.g., tungsten carbide) and metals are also suitable in certain embodiments. With the exception of semiconducting oxides, such as TiO₂ and ITO, semiconductor nanocrystals (often called quantum dots). Additional technology for the production of nanoparticles involves the use of dendrimers (highly branched synthetic polymers) or other polymers. The photosensitizer are typical attached to the dendrimer's surface or placed in the voids inside them for site targeting and controlled delivery, or a combination of targeting and detection. The nanoparticles may suitably be synthesized via colloidal synthesis and may take the form of colloidal crystals. Suitable nanoparticle precursors include polymerizable monomers preferably having 2, preferably more than 2, such as 3, 4 or 5 positions for intermolecular bonding, so as to form a network of interconnected precursors, that aggregate to form a nanoparticle.

The term "nanocarrier device" as used herein, refers to the inventive composition in the form of a nanoparticle, wherein the particle serves as a carrier for compounds such as photosensitizers, and optional imaging agents and targeting ligands.

The term "photosensitiser" as used herein, refers to a such compounds as chlorin e₆ (Ce₆), m-THPC, etc.

The term "nanophotomedicine" as used herein, refers to a photosensitizer complexed with the nanoparticles.

The term "doped nanophotomedicine" as used herein, refers to a nanophotomedicine doped with MR contrast agent and/or optical contrast agent.

The term "doped" as used herein, means that a small amount (about 1-15%) of another substance (in this case, a optical contrast agent and/or a magnetic contrast agent) has been added intentionally into the nanoparticle crystal.

The term "nanophotomedicine-conjugate" as used herein, refers to a nanophotomedicine conjugated with a targeting ligand.

The term "doped nanophotomedicine-conjugate" as used herein, refers to a doped nanophotomedicine conjugated with a targeting ligand and doped with Magnetic Resonance (MR) contrast agent and/or Optical contrast agent.

The present inventors have discovered a nanophotomedicine formulation that provides for improved efficacy in PDT treatment. The inventive nanophotomedicine formulation comprises a nanoparticle based on nanocrystals of metal sulphate, metal phosphate, metal oxide, or based on chitosan, polyvinylpyrrolidone (PVP), polylactic acid (PLA), polyethylenimine (PEI), poly(lactic-co-glycolic acid) (PLGA), or other suitable polymer and combinations thereof, such as a particle having a metal oxide core and a polymer shell, or a polymer core having a metal oxide shell, or any other combination of the above, including a ceramic construct, core or shell, wherein said nanoparticles, or shell or core thereof is doped with photosensitizer molecules, and wherein said photosensitizer molecules are distributed in said nanoparticles material in a quasi-aggregated state.

The term "quasi-aggregated state" is used herein to indicate that the photosensitizers are present in the nanocarrier (nanoparticle) at different levels of aggregation, that is, both in the form of aggregates as well as in the form of free photosensitizer (monomeric units).

In the present invention, a unique state of the photosensitizer drug within nanoparticles by a semi (quasi) aggregated state. This state can be defined by the enhanced absorbance of the photosensitizer in the Q band region of the UV-Vis spectrum as described herein, compared to its absorbance in the Soret band. More specifically, the ratio of the Q to Soret band absorbance, Q/S for the (stabilized) semi aggregated state is at least 0.3.

The Soret band is the main absorbance of any sensitizer in its monomeric form but it is in the blue region, where tissue penetration is low. Q band is a satellite band at the red-NIR region (having better tissue penetration) but always lower absorbance. Typically, Q/S is about 0.05 for monomers.

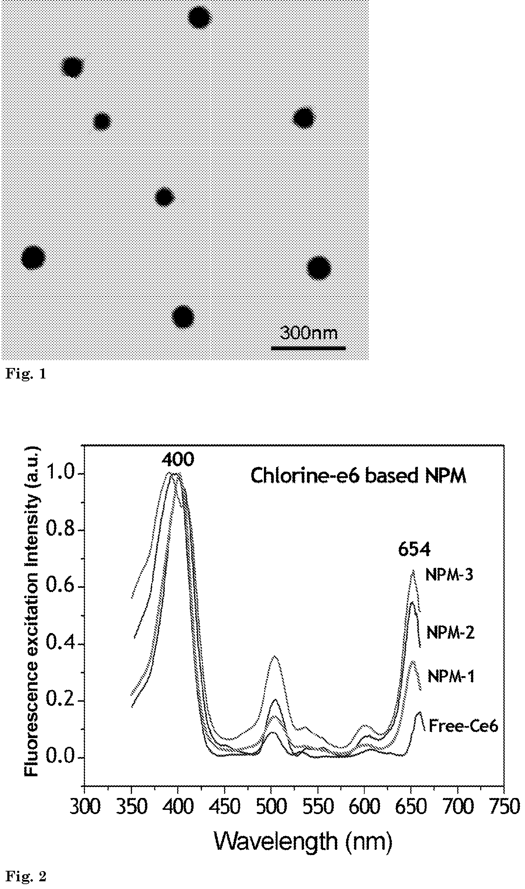

In the present invention a higher Q band absorption is attained by controlling the extent of amination (extent of linkage) of the sensitizer molecule with the nanoparticle matrix. For instance, as explained in more detail in the Examples below, NPM-1, is less aminated than NPM-2 which is in turn less aminated than NPM-3. As given in the examples of these specific NPMs, depending on the concentration of APTS and TEOS, and other reaction parameters, it is possible to obtain NPMs of different absorption levels in Q-band at 654nm by controlling the amount of aminated carboxyl groups of Ce6 (see Fig 2, NPM1-2 and 3 refers to different levels of absorbance at 654nm). For instance NPM-1 has a Q/S value of about 0.3, NPM-2 has a Q/S value of about 0.5 , NPM-3 has a Q/S value of about 0.7 and NPM-4 has a Q/S value of about 1 (the maximum, where Q band absorbance is maximized).

The present invention thus pertains to nanoparticles of metal sulphates, metal phosphates or (preferably) metal oxides or polymeric nanoparticles comprising a photosensitizer drug, said nanoparticles having Q/S value of at least 0.3. This value is irrespective of spectral peak maximum (the Q band having its maximum anywhere between about 600-900nm, and the S band having its maximum anywhere between 350-500nm).

The inventors have found that such a nanoparticle provides, amongst others, for highly improved photostability.

In preferred embodiments the nano-crystal is non-toxic and suitably luminescent.

To the outer surface of this composite architecture are preferably connected ligands targeted to cancer.

In another embodiment of the present invention, this photosensitizer-nanoparticle-targeting ligand conjugate overcomes *inter alia* the prior art problems of 1) aggregation/lipophilicity of photosensitizers; 2) low concentrations of photosensitizer per targeting ligand; 3) low absorption of photosensitizer at red region of visible spectrum; 4) non-specific accumulation of the photosensitizers; 5) uncontrolled aggregation of the photosensitizers molecule in blood circulation; 6) difficulties of dosimetry using fluorescence properties of photosensitizer itself; and 7) lack of non-invasive molecular image guided dosimetry, and pharmacokinetic estimation.

The surface chemistry of the nanoparticle conjugate is such that aggregation in physiological environments is avoided, that conjugates are not sequestered by the endoreticular system and that non-specific targeting of normal cells is minimized. It was also found that it is possible to load (very) high concentrations of photosensitizer within the nanoparticles to form a quasi-aggregated state. The amount of photosensitizer within the nanoparticles is suitably

Unlike the free photosensitizer in aggregated state, photosensitizer molecules in quasi-aggregated state absorb light efficiently which results in effective generation of reactive oxygen species. This result was unexpected and it may be explained by the specific conformation of the photosensitizer within the nanoparticle.

Without wishing to be bound by theory it is believed that the process of photosensitization within the nano-particle leads to controlled *in-situ* monomerization of drug and thereby controlled release of singlet oxygen over prolonged periods of time.

In addition to the photodynamic component of the nano-particle conjugates, the compositions of the present invention may additionally have incorporated optical contrast agents into (the core or shell of the) the nanoparticle. Suitable optical contrast agents include luminescent markers such as ZnS:Mn²⁺ QDs, fluorescent markers such as indocyanine green (ICG) and optical dyes such as methylene blue (MB). These markers allow for optical image guided, local drug delivery which provides a significant improvement of therapeutic efficacy for certain forms of cancers. The amount of optical contrast agents in the nanoparticles is suitably about 0.0001-15 wt%, preferably 0.0005-5 wt% based on the total weight of the doped nanoparticle.

In addition to the photodynamic component of the nano-particle conjugates, the compositions of the present invention may additionally have incorporated magnetic contrast agents into (the core or shell of the) the nanoparticle. This allows magnetic resonance imaging to be performed which provides a significant advantage in determining the systemic pharmacokinetics of the conjugates in clinical applications where the penetration of light limits the optical determination of their fate. The amount of magnetic contrast agents is suitably about 0.0001-15 wt%, preferably 0.0005-5 wt% based on the total weight of the doped nanoparticle.

As described above, the present invention leads to significant improvements in the current limitations of photosensitizer drugs and PDT. These improvements are due to the specific nature of the said nanophotomedicine formulation. One of the main advantages of this new nanoformulation, compared to conventional free-drug is that the photosensitive molecules are complexed with the carrier device in a quasi-aggregated fashion, a stage between monomers and aggregated molecules, i.e. where both monomers and aggregated molecules co-exist. Generally, in solution or in powder form, individual (monomeric) photosensitive dye molecules tend to aggregate, due to Van der Waals-like inter-molecular attractive forces. This aggregation is a critical obstacle in effective application of PDT because the fluorescence efficacy and singlet oxygen yield of the drug is significantly reduced in the aggregated state of the molecule.

Aggregation of photosensitizer drugs determined by the level of intermolecular interactions and is therefore a function of concentration of molecules in a solvent medium. Most of the photodrugs used in PDT are hydrophobic in nature and hence tend to aggregate under physiological conditions. Therefore, when using free drugs, only very low concentration can generally be used to maintain the monomericity of the photomolecules in circulation. On the other hand, the completely monomeric form of the drug also has the disadvantages of low absorption of red light that penetrates through tissue. Further, monomeric units undergo fast photobleaching leading to premature completion of the treatment due to the fact that the photomolecule concentrations drop below effective levels.

It has now been found that a trade-off between complete monomerization and aggregation can be achieved and that this provides for improved and prolonged PDT application.

Accordingly, the present invention provides a controlled complexation of the photodrug molecules to a nanocarrier device matrix (a nanoparticle matrix). This is achieved by providing a nanocarrier device with functional groups that can covalently bind individual photodrug molecules as monomeric units as well as quasi-aggregated species such that monomeric units co-exist with aggregated species separated by the functional groups in the nanoparticle matrix. One suitable manner in which this can be achieved is to prepare a nanoparticle from photosensitizer-conjugated nanoparicle precursors. In the architecture that is thus attained, the monomeric units upon laser irradiation start releasing singlet oxygen species and part of the singlet oxygen actively disintegrates quasi-aggregated clusters of photodrug molecules giving rise to new monomeric units. Consequently, that there will be a continuous supply of photoactive monomeric units for long duration of laser therapy. Most importantly, the nanoparticle formulation of the present invention is physico-chemically stable in solid state (powder form) or in aqueous / bio-chemical medium and hence the photophysical properties remain largely unaltered under physiological conditions. This has the advantage that aggregation of the drug in the blood and associated pharmacokinetics issues of the drugs are absent.

Another advantage of the present invention is that the unique architecture of the nanophotomedicines and complexation of the photodrug leads to a significant improvement of photoabsorption of the photodrug in the red and near-infrared region of the visible light spectrum where the tissue penetration of light radiation is higher. This property has significant importance in improving the efficacy of phototherapy because most of the free photodrug molecules have minimum absorption in red region (viz. Q band) compared to ultraviolet or blue region (Soret-band) of electromagnetic spectrum. This limits the use of free photodrugs, as drugs need to be photosensitized all throughout the interior region of the tumor using light radiation with high tissue penetration such as red light. Improvement in the absorption property of the photodrug in the red region, viz. Q-band is therefore needed. Accordingly, the present invention provides a nanoformulation of photodrugs wherein the photo-absortion is significantly higher in the Q-band, many times as high as that of Soret band. This improved absorption property is unique to the said nanoformulation achieved by way of controlled supramolecular interaction of the quasi-aggregated drug molecules with that of nanocarrier device.

Yet another important feature of the present invention is related to the higher stability of the photomedicine within the nanocarrier device resulting in prolonged release of cytotoxic singlet oxygen. Generally monomeric free photodrugs, particularly the hydrophilic molecules like chlorin e₆, undergo rapid photobleaching due to the attack of singlet oxygen produced by the molecule itself. This limits the availability of sufficient concentrations of photodrug at the diseased site and hence limits the therapeutic efficacy of the drug in damaging the cancer. Direct modification of the molecules to stabilise against photobleaching may affect the quantum yield of singlet oxygen production and is not desirable. It is therefore important to prepare a photodrug formulation in which the singlet oxygen yield is maintained and which at the same time will exhibit less photobleaching.

Accordingly, the present invention provides a nanophotomedicine formulation wherein the monomeric units of the drug are not exposed to the bleaching effect of full laser light. Instead, the photodrug is complexed together with the nanocarrier matrix as a stable mixture of monomeric units and quasi aggregated units, such that upon laser irradiation the singlet oxygen produced by the monomers cause de-aggregation of quasi-aggregated units so as to provide a continuous supply of cytotoxic concentration of singlet oxygen even for long durations of irradiation and/or high photodose.

Yet another advantage of certain embodiments of the present invention is the capability of nanophotomedicines to provide magnetic and optical contrast imaging of the diseased site prior to or during the phototherapy. Image-guided radiation therapy is an emerging area in the clinical practice where the exact location, size and spread (angiogenesis/metastasis) of cancer is detected and used to direct radiation therapy. This is achieved by aligning the actual imaging coordinates of the drug in the body, as revealed by computed tomography or MRI, with the irradiation treatment plan prior to and during the therapy. This kind of image assisted phototherapy has major advantage in effective cancer management. Accordingly, the possibility to provide the nanophotomedicine of the invention with an optical marker and/or magnetic contrast agent and to use the thus doped nanophotomedicine together with therapeutics is an important aspect of this invention.

In yet another embodiment of aspects of the present invention the nanophotomedicine construct is provided with the property of specifically targeting the diseases sites such as cancer. This can be achieved by providing the nanophotomedicine surface with targeting moieties such as receptor-ligands. This helps to achieve targeted photodynamic therapy of cancer. The amount of targeting ligand is suitably about 0.00001-1 wt%, based on the total weight of the nanoparticle.

To prove this concept the present inventors have prepared nanophotomedicine comprising a photosensitizer, a nanoparticle and a targeting ligand. As the photosensitizer drugs, meta-tetrahydroxyphenylchlorin (m-THPC/Foscan) and chlorin e₆ (Ce₆) were chosen, as nanoparticle a nanoparticulate silica was chosen, and as the targeting ligand octreotide was chosen. Octreotide is a synthetic analogue of somatostatin. Many neuroendocrine tumors and (activated) immune cells express a high density of somatostatin receptors (sst). The skilled person will understand that variations in the selection of the photosensitizer, the nanoparticle and the targeting ligand can be made. The inventors have used the thus prepared targetable nanophotomedicine in experimental setups in various aqueous media and *in vitro* in sst positive (K562 cells, human myeloid cell line) as well as in wild-type cells to confirm the validity of the approach. *In vitro* absorption and excitation spectroscopy of the conjugate combined with singlet oxygen quantum yield data and cell proliferation assays as described in the Examples below confirm that these nanophotomedicines exhibit the desired therapeutic efficacy. It is important to note that the present inventors envisage that similar approaches can be used to target other receptors and that the choice of photosensitizer and nanoparticle is not critical.

The present invention provides a novel nano-photomedicine that can potentially target cancer cells *in vivo,* enhance tumor contrast by bi-modal fluorescent and magnetic resonance imaging and destroy cancer cells by controlled delivery of reactive oxygen species under visible light exposure.

A characterizing feature of the present invention is the conjugation of photosensitizer with nanoparticles in a desired quasi-aggregated fashion and the discovery that this changes the physio-chemical properties of photosensitizer to make it suitable for effective and targeted PDT. The discovery that this conjugation produces a quasi-aggregated state of photosensitizer that can better absorb light at wavelengths that penetrate tissue compared to the free, non-conjugated drug and allow for the controlled release of reactive oxygen species upon irradiation was not anticipated. It is a particular advantage that the present therapeutic composition can be combined with a targeting ligand and magnetic contrast functionality as this supports image assisted PDT applications.

### Methods for producing the nanophotomedicine of the invention

The nanophotomedicine of the invention can be prepared in many different ways. Depending on the type of nanoparticle and on the chemistry of the photosensitizer and the nanoparticles matrix (the material for preparation of the nanoparticles).

It is essential that the photosensitizer is provided associated with the nanoparticle in a quasi-aggregated state. As will be shown herein, suitable nanoparticles can be obtained by precipitation of a precursor material into nanocrystals from solution at low temperature (e.g. 20-80°C) or by a high temperature (thermal) process. Preferably the nanoparticle is obtained by precipitation from a solution or from a colloid. Suitable precursor materials that under suitable conditions will precipitate to form a nanoparticle include, but are not limited to, metal sulphides, metal phosphates and metal oxides and combinations thereof, such as silicates and calcium phosphates. A highly preferred method is that known in the art for preparing colloidal silica particles. The particles may be amorphous crystals or fully crystallized. The metal sulphide, metal phosphate and/or metal oxide particles may be used plain or may be covered or combined a polymeric material to provide a ceramic. The nanoparticles of the present invention are doped with photosensitizers, luminescent materials, and magnetic materials preferably by inclusion during the formation of the particle.

The photosensitizer is preferably covalently bonded to the nanoparticle. In the case of silica, a silicate-reactive photosensitizer is therefore suitably (and preferably) produced. Very suitable, a silane coupling agent is used as a crosslinking agent between the photosensitizer and the silicate. Very suitable, aminopropyl triethoxysilane (APTS) is used, as such a compound can be reacted with a silicate to provide the silicate with an amine-functionality.

One step in the method of producing a nanophotomedicine of the invention, in an embodiment wherein the nanoparticle is silicate, is the provision of an amine-reactive photosensitizer. For this, a carboxyl-containing photosensitizer (Ce₆ has three carboxyl groups per molecule) is suitably activated by reacting the photosensitizer with a molar excess of a carbodiimide such as EDC (EDAC), preferably in the presence of a succinimide such as Sulfo-NHS, usually in the solvent DMSO. In this reaction, the carboxyl groups on the photosensitizer are activated to form amine-reactive intermediates, such as amine-reactive Sulfo-NHS esters. The activation reaction is suitably allowed to continue for about 1-10 hrs, usually about 4 hrs to provide the amine-reactive photosensitizer. The product is optionally purified by gel-filtration.

A second step in the production in such an embodiment is suitably the reaction of the amine-reactive photosensitizer with aminopropyl triethoxysilane (APTS) to provide a silicate-reactive photosensitizer (a functionalized photosensitizer). This coupling reaction is suitably continued for 3-4 hrs in dark, at room temperature. One example of a compound resulting from this step is Ce₆-APTS.

In the next step, the silicate-reactive photosensitizer, e.g. Ce₆-APTS, is reacted with an orthosilicate precursor for the synthesis of nanostructured silica powders by sol-gel processes such as tetraethyl orthosilicate (tetraethoxysilane, TEOS), and tetramethyl orthosilicate (tetramethoxysilane, TMOS) to provide a photosensitizer-conjugated orthosilicate (e.g. Ce₆-TEOS or Ce₆-TMOS). This conjugation reaction is suitably performed for a duration of 2-3 hrs in 99% ethanol. The photosensitizer-conjugated orthosilicate forms the precursor for nanoparticle device wherein the silane-coupled quasi-aggregated photosensitizer is embedded to form the nanophotomedicine of the invention. The inventive nanoparticles are achieved by using these photosensitizer-conjugated orthosilicate precursors in a sol-gel reaction.

The initial stages in the sol-gel reaction involve obligatory hydrolysis of the orthosilicate precursor, and condensation of the hydrolyzed products to form small (3-4 silicon) particles, which aggregate to form the larger colloidal silica particles that may eventually condense to form a silica gel. However, this latter stage is preferably not part of the process of the present invention. The particles have a final size of about 90-100nm and in the absence of acidic conditions do usually not condense to form gels. Hydrolysis and condensation of the photosensitizer-conjugated orthosilicate precursors (e.g. the Ce₆-conjugated TEOS and/or TMOS precursors) produces nanosized silica powders wherein the photosensitizer is covalently bonded in the silica matrix.

The hydrolysis of the Ce₆-conjugated TEOS and/or TMOS precursors may be achieved in aqueous solution by adding to the ethanol medium a small amount of water and a strong base such as NH₄O₄ or other ammonium source or NaOH. Next, this aqueous solution is sonicated, for instance for a duration of 10 minutes using an interval of 2 minutes, which sonication results in the precipitation of nanoparticles of quasi-aggregated photosensitizer complexed within the silica matrix. The thus precipitated nanophotomedicine particles may then be separated from the aqueous medium (usually an ethanol/water/ammonium mixture) by centrifugation, and may optionally be washed in water and re-dispersed into PBS or water.

Although in the above example an APTS-modified silicate precursor could in principle be reacted with an amine-reactive photosensitizer, it is preferred that the APTS-coupled photosensitizer is reacted with the silicate precursor, as this results in the photosensitizers being incorporated into the growing nanoparticle in the more favourable quasi-aggregated state.

Thus, preferably, a photosensitizer is provided with functional groups for the covalent attachment to nanoparticle precursors so as to provide a functionalized photosensitizer. The skilled person is well aware of the various possibilities of attaching molecules such as photosensitizers to nanoparticle precursors. These techniques generally involve the introduction of amino-, silane-, thiol-, hydroxyl- and/or epoxy-functionalities to the photosensitizers, and the subsequent attachment thereto of the nanoparticle precursor, optionally using cross-linkers. When referring to such an embodiment of an aminoalkylsilanization of a photosensitizers in more general terms, a method of preparing a functionalized photosensitizer for covalent binding to a nanoparticle precursor according to one embodiment of the present invention may also be described as to employ bifunctional monomers that act as linking agents and link the photosensitizer to the nanoparticle precursor.

Very suitably, the bifunctional monomer may have two different chemical functionalities, such that one functionality is capable of reacting with the nanoparticle precursor and the other is capable of reacting with a functionalized group of the photosensitizer.

The functionalized photosensitizer and nanoparticle precursors are mixed in solution or suspension under conditions that i) allow for the covalent bonding of the photosensitizer to the nanoparticle precursor to form a photosensitizer-conjugated nanoparticle precursor, and that ii) allow the formation of nanoparticle precursor complexes via intermolecular bonding of said nanoparticle precursors and aggregation of said nanoparticle precursors complexes to form the nanoparticle by subsequent steps as condensation and aggregation. Preferably, step i) is allowed to occur before step ii). This results in the situation wherein the photosensitizer is provided associated with the nanoparticle in a quasi-aggregated state.

The nanoparticles to which the photosensitizer is conjugated may during or after its preparation additionally be doped with luminescent markers and/or magnetic contrast markers. This is preferably performed as described below.

### Luminescent quantum dot-doped nanophotomedicine

The method of doping the nanoparticles with luminescent materials may generally be performed as follows. First the photosensitizer-conjugated nanoparticle precursor is prepared as described above. The luminescent marker is doped within the nanomatrix during the hydrolysis and condensation of this precursor by the addition of the marker to the hydrolysis and condensation solution. Then conditions for the formation of nanoparticle precursor complexes via intermolecular condensation of said nanoparticle precursors and aggregation of said nanoparticle precursors complexes to form the nanoparticle are applied. In the case of orthosilicate precursors, these include the provision of for instance 1-5 % of NH₄O₄. A suitable amount of the luminescent marker is for instance 0.01 µM ZnS:Mn²⁺ in the precipitate solution. Sonication for 10 minutes leads to the precipitation of nanoparticles of silicondioxide complexed with quasi-aggregated Ce₆ and ZnS:Mn²⁺ QDs, which remain embedded within the nanoparticulate matrix. Precipitated doped nanophotomedicine are separated from the medium by centrifugation and are preferably washed and stored in PBS. For administration of the nanophotomedicines of the invention, the devices are preferably suspended into PBS.

### Magnetic contrast agent-doped nanophotomedicine

The method of doping the nanoparticles with magnetic contrast agent is essentially the same as described above for the luminescent marker. First the photosensitizer-conjugated nanoparticle precursor is prepared as described above. The precursor for magnetic contrast agent, for example: 0.001-10% Gd³⁺ (GdNO₃) or 0.001-10% (Mn²⁺) MnCl₂, or 0.001-10% Fe³⁺ (FeCl₃) is added to the hydrolysis and condensation solution which forms the nanoparticles. The use of Gadolinium nitrate, for instance, results, under appropriate conditions, in the precipitation of nanoparticles complexed with quasi-aggregated photosensitizer and doped with Gd³⁺, which remains embedded within the amorphous phase of nanoparticulate matrix. The precipitated nanoparticles are separated from the medium by centrifugation and are preferably washed before use.

### Applications of the invention.

Targeted therapy is a central goal of medicine and minimizing damage to normal tissue surrounding a target treatment volume is critically important. PDT can be applied in practically any location in the body. While PDT induces a systemic immune response following illumination, the radius of action of reactive oxygen species is very much smaller than the radius of a single cell. In practice photosensitizer conjugates do not exhibit dark toxicity, therefore, local selectivity (within the light field) offers significant opportunities. No cells or tissues are resistant or have been shown to develop resistance to high concentrations of reactive oxygen species. The present invention can overcome many of the significant disadvantages of the prior art targeted photodynamic therapy. Where photosensitizer conjugates have limited bioavailability, non-specific uptake and a limited capacity for generating reactive oxygen species per targeting ligand. As described herein, sst2 expressing cells were used to prove the principle of the approach of the present invention. The present invention can be used to target other receptors and can be applied with other photosensitizers. Molecular targets for cancer are widespread and specific to the type of tumor. There is a clear rational for investigating receptor targeted PDT of breast, prostate, lung, brain tumors and for cancer in the GI tract. Other non-malignant conditions can also be targeted. For example, since activated immune cells in the affected joints of patients with rheumatoid arthritis express a high density of somatostatin (SS) receptors (sst). Targeted PDT would be an ideal candidate for treating this condition.

The present invention will now be explained in more detail by way of the following non-limiting Examples.

### EXAMPLES

The following examples describes the method of making nanophotomedicines (NPM) with two separate, representative photosensitizers, viz. chlorin e₆ (Ce₆) or mTHPC are covalently embedded within the nano-sized (50-150nm) carrier device of silica or chitosan in a suitable quasi-aggregated stage of desired photo-absorption property of the final construct at the Q-band (Red-NIR) region where the tissue penetration of light is better. Doping of these nanophotomedicines with the second component of luminescent quantum dots suitable for optical imaging and third component of paramagnetic ions suitable for MRI contrast imaging to form a doped nanophotomedicine and/or conjugation with active cancer targeting peptide ligand to form a (doped) nanophotomedicine conjugates, novel photobleaching characteristics, delivery to cancer cells, and photodynamic therapy using the said nanophotomedicine are described in separate examples.

The reagents used for the preparation of nanophotomedicine included tetraethyl orthosilicate (TEOS , Sigma 98%) or tetramethyl orthosilicate (TMOS), aminopropyl triethoxysilane (APTS, Sigma 98%), ammonia (25% solution, Sigma Aldrich), 1-ethyl-3- [3-dimethylaminopropyl] carbodiimide hydrochloride (EDAC or EDC), N-hydroxysulfosuccinimide (Sulfo-NHS), N,N'-disuccinimidyl carbonate (DSC, Sigma, 98%), ethanol (99%, Sigma), 2-(N-morpholino)ethanesulfonic acid (MES) buffer (Sigma), phosphate-buffered saline (PBS), chlorin e₆, m-tetrahydroxyphenylchlorin (mTHPC), ZnS:Mn quantumdots (QDs), Gadolinium (Gd³⁺)-nitrate (99%, Sigma), and dimethyl sulfoxide (DMSO, Sigma), all analytical grade reagent and were used without further purification.

In the method of synthesis, unlike the prior art (US patent No. 7364754) we used surfactant-free non-micellar medium containing simple, low-cost and homogeneously miscible solvent system of ethanol or DMSO with water.

### Example 1: Production of nanophotomedicine NPM-1 using Ce₆ as photosensitizer

In this example preparation of photsensitizer chlorin e₆ (Ce₆) based nanophotomedicine (viz. NPM-1) having ∼ 2 fold higher absorption of light by the final construct in the Q-band (654 nm) region compared to that of free Ce₆ is presented.

A 1 µM concentration of Ce₆ (commercially available from for instance Porphyrin Products, Logan, UT) was reacted with 10-15 fold molar excess of EDAC and 10-15 molar excess of Sulfo-NHS in 5 ml of 99% DMSO. After 4 hrs of reaction, the conjugated product is purified by gel-filtration providing amine reactive photosensitizer, which is further reacted with the silane coupling agent using 200 µL of APTS. The coupling reaction is continued for 3-4 hrs in dark, at room temperature, providing the compound Ce₆-APTS. In the next step, the Ce₆-APTS is reacted with 600 µL (about 600 mg) of TEOS or TMOS for 2-3 hrs in 10ml of 99% ethanolic medium, forming the precursor for silane-coupled quasi-aggregated photosensitizer. Hydrolysis of this precursor by the addition of 3 ml of water and 600 µL NH₄O₄ under sonication for 10 minutes with an interval of 2 minutes leads to the precipitation of nanoparticles of quasi-aggregated Ce₆ complexed within silica matrix. The precipitated NPM-1 are separated from the solvent medium by centrifugation (6000 rpm, 5 minutes) and washed with distilled water before being re-dispersed into PBS.

Transmission electron micrograph (Fig. 1) shows the formation of uniform spherical nanoparticles of size 90-100nm. The fluorescent excitation spectrum of the NPM-1 (Fig. 2a) shows that the absorption of light at 654nm corresponding to triplet-state electronic transition responsible for singlet oxygen generation and/or fluorescence of the construct is ∼ 2 fold higher than that of free-photosensitizer. This indicates that the photosensitizer within the nanophotomedicine construct is no longer in the free form but instead is a complexed entity covalently connected by amide linkage mediated by aminopropyl silane groups.

### Example 2 : Characteristics of nanophotomedicine NPM-2 with Ce₆ as photosensitizer

In this example, processing of nanophotomedicine (NPM-2) with ∼ 4 fold higher absorption of light in the Q-band compared to that of free Ce₆ is illustrated.

A 1 µM concentration of Ce₆ was reacted with 10-15 fold molar excess of EDAC and 10-15 molar excess of Sulfo-NHS in 5 ml of 99% ethanol. After ∼ 4 hrs of reaction, the conjugate is purified by gel filtration providing amine reactive photosensitizer which is reacted with the silane coupling agent using 300 µL of APTS. The coupling reaction is continued for 3-4 hrs in dark, at room temperature, providing the compound Ce₆-APTS. In the next step, Ce₆-APTS is reacted with 800 µL of TEOS or TMOS for 3 hrs in 10ml of 99% ethanolic medium, forming the precursor for NPM-2. Hydrolysis of this precursor by the addition of 3 ml of water and 600 µL NH₄O₄ under sonication for 15 minutes with an interval of 2min leads to the precipitation of NPM-2 nanophotomedicines wherein Ce₆ is quasi-aggregated in still higher level and remains covalently embedded within the nanoparticulate matrix through amide linkage. Precipitated NPM-2 particles are separated from the ethanolic medium by centrifugation and are washed with distilled water before being re-dispersed into PBS. The fluorescent excitation spectra of NPM-2 shown in Fig.2 indicate a ∼ 4 fold increase in the absorption of the Q-band at 654 nm compared to that of free drug. The absorption in the Soret band region remains largely unchanged. This enhanced absorption of Q-band can leads to production of singlet oxygen at higher tissue depth compared to the case of free photosensitizer.

### Example 3: Production of nanophotomedicine NPM-3 using Ce₆ as photosensitizer

In yet another example, the production of nanophotomedicine (NPM-3) with ∼ 7 fold higher absorption in the Q-band region compared to that of free Ce₆ is illustrated.

A 1 µM concentration of Ce₆ was reacted with a 10-15 fold molar excess of EDAC and a 10-15 molar excess of Sulfo-NHS in 5 ml of 99% ethanol. After 4 hrs of reaction, the conjugated product was purified by gel filtration providing amine reactive Ce₆ which is reacted with the silane coupling agent using 600 µL of APTS. The coupling reaction was continued for 3-4 hrs in dark, at room temperature, providing the compound Ce₆-APTS. In the next step, the Ce₆-APTS was reacted with 1000 µL of TEOS or TMOS for 2-3 hrs in 10 ml of 99% ethanolic medium, forming the precursor for silane coupled quasi-aggregated photomedicine. Hydrolysis of this precursor by the addition of 3 ml of water and 800 µL NH₄O₄ under sonication for 20 minutes with an interval of 2 minutes leads to the precipitation of NPM-3 nanoparticles complexed with further quasi-aggregated Ce₆, which remain embedded within the nanoparticulate matrix. The precipitated NPM-3 particles are separated from the ethanolic medium by centrifugation and are washed with distilled water before being re-dispersed into PBS solution for administration.

The fluorescent excitation spectra of NPM-3 (Fig. 2) indicates still higher absorption of light at the 654 nm region, nearly 7 fold higher than that of the free drug and ∼ 75% equivalent to that of Soret band absorption of the same construct.

The controlled increase in the absorption of Q-band of the nanophotomedicine by the extent of chemical modification and most importantly stabilization of the same within the nanocarrier device that protect the photosensitizer molecule from any further uncontrollable aggregation in water or PBS or due to the influence of proteins in the blood or after accumulation in diseased site is an important achievement of the present invention. Thereby, the present invention overcomes one of the greatest challenges of uncontrolled aggregation of photosensitizer and loss of photosensitive properties observed with free photosensitizers.

### Example 4: Production of nanophotomedicine NPM-4 using mTHPC as photosensitizer

In this example, the production of nanophotomedicine (NPM-4) with another important photosensitizer mTHPC is illustrated. The product shows a 100% shift of light absorbance properties from the Soret to Q band at 652nm while maintaining its high fluorescence and photosensitizer activity.

A 1 µM concentration of amine-reactive mTHPC was treated with 600 µL silane coupling agent APTS for 24 hrs in the dark. After 24 hrs, the mTHPC-APTS conjugate was reacted with 1000 µL of TEOS or TMOS for 6 hrs in 10ml of 99% ethanolic medium, forming the precursor for silane-coupled quasi-aggregated nanophotomedicine, mTHPC. Hydrolysis of this precursor by the addition of 6 ml of water and 800 µL NaOH under sonication for 20 minutes with an interval of 2 minutes leads to the precipitation of NPM-4 nanoparticles complexed with quasi-aggregated mTHPC.

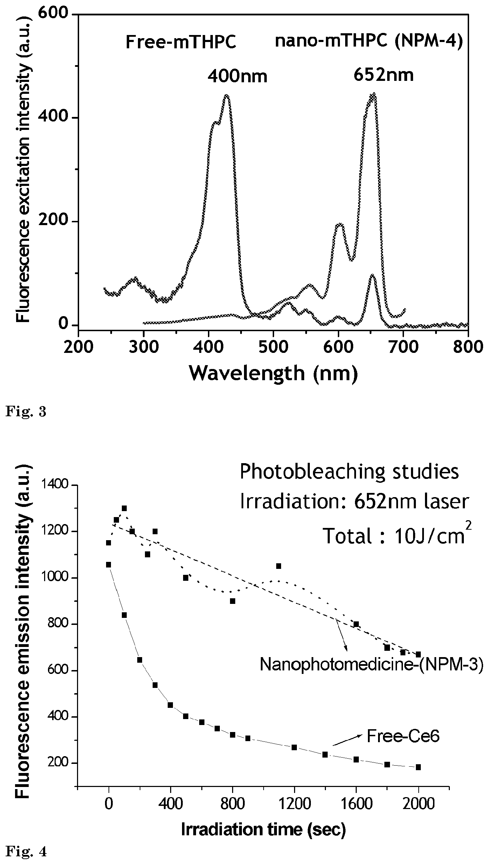

This product shows completely different absorption/excitation characteristics compared to free-mHPC, as shown in Fig. 3. The absorption at Soret band ∼ 400nm was found completely quenched whereas the essential absorption needed for phototherapy, at the Q-band was enhanced by 70-80%. The Q-band absorption which is as high as that of Soret band of free-sensitizer can result in a significant enhancement of the therapeutic efficacy during photodynamic therapy. This construct overcomes one of the major disadvantages of free photosensitizer, that is low-light absorption in the red region of the spectrum

### Example 5: Ex vivo photophysical properties of nanophotomedicine NPM-3

In this example, the photophysical properties of the nanophotomedicine prepared in Example 3 (NPM-3) is illustrated. Significant improvements of the product in comparison to the free drug in photodynamic therapy are demonstrated.

Photostability of the drug is very important for extended therapy of disease like cancer. However, photodrugs, particularly water soluble drugs like Ce₆ undergo very fast photodegradation as it is subject to degradation by singlet oxygen produced by the drug itself. This leads to premature completion of the treatment due to an insufficient concentration of the drug at the disease site. In this example it is shown how nanophotomedicine overcomes this problem.

Photobleaching characteristics of free Ce₆ and nanophotomedicine (NPM-3) having nearly the same initial florescence intensity (that correlate with concentration of the drug) is compared using a fluorescence spectrometer. Laser irradiation of samples of both products was carried out under identical condition of a total dose of 10 J cm⁻². Fig.4 illustrates the changes in the fluorescence emission characteristics of both the samples. Free Ce₆ shows typical fast bleaching which results in an inactivation of the drug at intensities as low as 2.5 J cm⁻², whereas the NPM-3 construct shows a unique non-linear characteristic and photostability of the embedded drug even after receiving a photodose of 10 J cm⁻². The photobleaching curve for NPM-3 shows multiple phases involving both enhancement and reduction of the fluorescence emission from the drug. This reveals a spatially heterogeneous (quasi-aggregated) nature of the embedded drug upon interaction with light and indicates that the drug is subject to *in situ* monomerization followed by bleaching in a repeated fashion. In effect, this leads to long term stability of the drug within the construct even after extended duration of therapy.

### Example 6 In vivo photophysical properties of nanophotomedicine NPM-3

In this example, the photostability of nanophotomedicine intracellularly within of cancer cells was tested and compared to that of free photosensitizer.

Leukemia cells K562 were seeded at 800.000 cells / well in a 12 well tissue culture plate and treated with both free Ce₆ (1 µM) and nanophotomedicine (NPM-3) prepared by using the same concentration of the sensitizer. Cells were incubated at 37 °C for 3 hrs before imaging using confocal microscope. Fluorescence imaging was carried out by exciting the sensitizer or nanophotomedicine taken-up by the cells using 405 nm laser. For recording the photobleaching at intracellular regions of the cancer cells, which has significant correlation to therapeutic effects, imaging is carried out after stipulated duration of laser irradiation from 1-360 seconds).

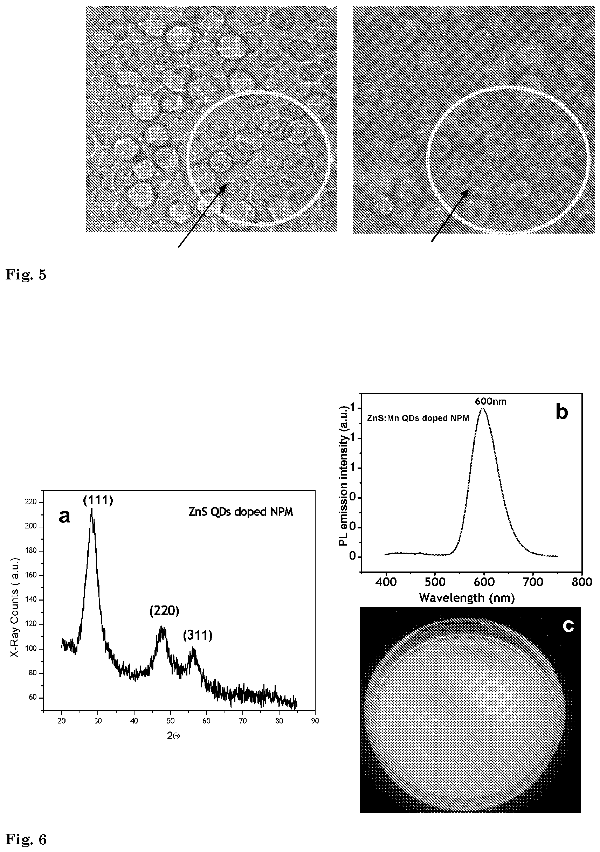

Fig. 5ashows confocal images of cells treated with free-Ce6 wherein the drug was found completely bleached out in the region of laser irradiation after 30 seconds whereas in Fig. 5b, cells treated with nanophotomedicine, showed stable fluorescence even up to 360 seconds. This confirms that the spectroscopic characteristics observed as described in Example 5 is also true in biological cells, i.e. *in vivo.* This unique character of the nanophotomedicine is critical in providing extended duration of phototherapy of cancer as the maintenance of fluorescence activity of the drug is essential for phototherapy.

### Example 7: Production of luminescent quantum dot-doped nanophotomedicine

In this example, the production of nanophotomedicine NPM-5, doped with luminescent quantumdots of ZnS:Mn2+ is illustrated to form a doped nanophotomedicine.

Luminescent QDs are promising candidates for in vivo imaging of disease including cancer. However, luminescent QDs used usually contain the toxic heavy-metal cadmium in the composition (CdS, CdSe, CdTe, etc). This limits the use of such QDs as well as nanodevices doped with such QDs for human clinical applications. In contrast, the present invention use a completely non-toxic quantum dots based on ZnS doped with metals (Cu or Al) or transition metals (Mn) for incorporation into the nanophotomedicine, which can be used for optical imaging of the NPMs *in vivo* without affecting the fluorescence and the singlet oxygen generating properties of the embedded photosensitizer.

Accordingly, in a typical preparation, 1 µM Ce₆ reacted with 10-15 fold molar excess of EDAC and 10-15 molar excess of Sulfo-NHS in 5 ml of 99% ethanol. After 2-4 hrs of reaction, the conjugated product is purified by gel filtration resulting amine reactive 'activated' photosensitizer to react with the silane coupling agent 600 µL of APTS. The reaction is continued for 3-4 hrs in dark, at room temperature, providing the compound Ce₆-APTS-1. After 3-4 hrs, the Ce6-APTS-1 is reacted with 1000 µL of TEOS or TMOS for 2-3 hrs in 10ml of 99% ethanolic medium, forming the precursor for silane coupled quasi-aggregated photosensitizer. The quantumdots are doped within the nanomatrix during the hydrolysis and condensation of this precursor by the addition of 3 ml of water containing 0.01 µM ZnS:Mn2+ QDs and 800 µL NH₄O₄ under sonication for 10 minutes leads to the precipitation of nanoparticles of silicondioxide complexed with quasi-aggregated Ce₆ and ZnS:Mn₂+ QDs, which remain embedded within the nanoparticulate matrix. Precipitated doped nanophotomedicine is separated from the ethanolic medium by centrifugation and is washed with distilled water before being re-dispersed into PBS solution for administration.

Fig.6a shows the X-ray diffraction pattern of embedded ZnS QDs within the nanophotomedicine, Fig. 6b and Fig. 6c shows the fluorescence emission spectra at 600nm and digital photograph of water dispersed sample emitting orange color from embedded ZnS:Mn confirming the successful doping of QDs within the doped nanophotomedicine.

The fluorescence emission from the QDs can be used for imaging the cancer in vivo, after localization of the doped nanophotomedicine at targeted tissue, using fiber optic excitation and emission devices. This help to improve the current methods of photodynamic dosimetry which currently rely on the fluorescence properties of the free photosensitizer. The use of QDs for cancer detection and dosimetry helps to achieve these goals without photobleaching (destroying) the photosensitizer.

### Example 8: Production of gadolinium (Gd3+)-doped nanophotomedicine

In this example, the production of NPM-5 doped with magnetic contrast agent of gadolinium (Gd3+) to form a doped nanophotomedicine, is described.

A 1 µM concentration of Ce₆ was reacted with 10-15 fold molar excess of EDAC and 10-15 molar excess of Sulfo-NHS in 5 ml of 99% ethanol. After 2-4 hrs of reaction, the conjugated product is purified by gel filtration resulting amine reactive 'activated' photomedicine to react with the silane coupling agent 600 µL of APTS. The reaction is continued for 3-4 hrs in dark, at room temperature, providing the compound Ce₆-APTS-1. After 3-4 hrs, the Ce₆-APTS-1 is reacted with 1000 µL of TEOS or TMOS for 2-3 hrs in 10ml of 99% ethanolic medium, forming the precursor for silane coupled quasi-aggregated nanophotomedicine. The magnetic agents are doped within the nanomatrix during the hydrolysis and condensation of this precursor by the addition of 3 ml of water containing 0.01M gadolinium nitrate followed by 800 µL NH₄O₄ under sonication for 10 minutes leading to the precipitation of nanoparticles of silicondioxide complexed with quasi-aggregated Ce₆ and doped with Gd³⁺, which remain embedded within the amorphous phase of the nanoparticulate matrix. The precipitated nanoparticles are separated from the ethanolic medium by centrifugation and are washed with distilled water before being re-dispersed into PBS solution.

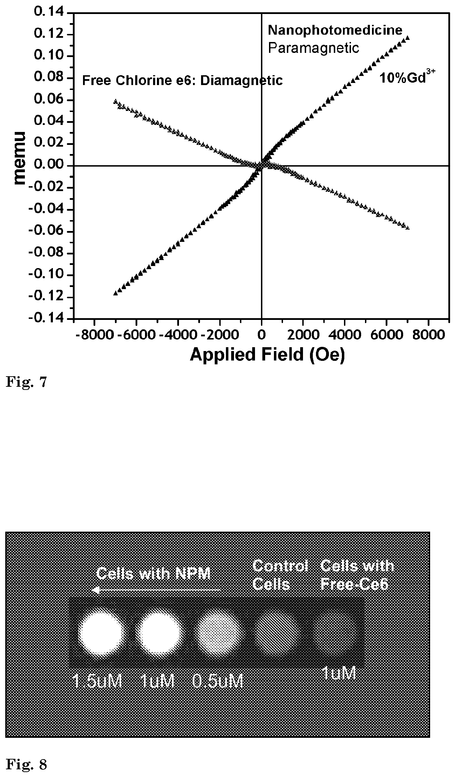

Magnetic studies carried out using vibrating sample magnetometer revealed the paramagnetic property (Fig.7) of the Gd³⁺ doped nanophotomedicine compared to the diamagnetic response of free Ce₆. Further, the applicability of this system for magnetic resonance imaging was demonstrated by imaging a collection of ∼ 80.000 cancer cells treated with the doped nanophotomedicine in a 24 well plate using a clinical MRI unit at 1.5T. Fig. 8 shows the T1 weighed contrast imaging of the cells treated with the doped nanophotomedicine of different concentration together with control (untreated cells) and free Ce₆ treated cells. It can be seen that the contrast increases with the concentration of nanophotomedicines, confirming that the doped nanophotomedicines described in the present invention can be used for MRI based diagnosis together with photodynamic therapy. This has significance in pre-therapeutic planning, understanding the pharmacokinetics of the administrated drug using completely non-invasive technique and post-treatment efficacy analysis.

### Example 9: Production of Manganeze (Mn2+)-doped nanophotomedicine

In this example, the production of NPM-6 doped with magnetic contrast agent of manganese (Mn²⁺) to form a doped nanophotomedicine, is described.

A 1 µM concentration of Ce₆ was reacted with 10-15 fold molar excess of EDAC and 10-15 molar excess of Sulfo-NHS in 5 ml of 99% ethanol. After 2-4 hrs of reaction, the conjugated product is purified by gel filtration resulting amine reactive 'activated' photomedicine to react with the silane coupling agent 600 µL of APTS. The reaction is continued for 3-4 hrs in dark, at room temperature, providing the compound Ce₆-APTS-1. After 3-4 hrs, the Ce₆-APTS-1 is reacted with 1000 µL of TEOS or TMOS for 2-3 hrs in 10ml of 99% ethanolic medium, forming the precursor for silane coupled quasi-aggregated nanophotomedicine. The magnetic agents are doped within the nanomatrix during the hydrolysis and condensation of this precursor by the addition of 3 ml of water containing 0.01M manganese sulfate followed by 800 µL NH₄O₄ under sonication for 10 minutes leading to the precipitation of nanoparticles of silicondioxide complexed with quasi-aggregated Ce₆ and doped with Mn²⁺, which remain embedded within the amorphous phase of the nanoparticulate matrix. The precipitated nanoparticles are separated from the ethanolic medium by centrifugation and are washed with distilled water before being re-dispersed into PBS solution.

### Example 10: Production of Iron (Fe3+)-doped nanophotomedicine

In this example, the production of NPM-5 doped with magnetic contrast agent of Iron (Fe³⁺) to form a doped nanophotomedicine, is described.

A 1 µM concentration of Ce₆ was reacted with 10-15 fold molar excess of EDAC and 10-15 molar excess of Sulfo-NHS in 5 ml of 99% ethanol. After 2-4 hrs of reaction, the conjugated product is purified by gel filtration resulting amine reactive 'activated' photomedicine to react with the silane coupling agent 600 µL of APTS. The reaction is continued for 3-4 hrs in dark, at room temperature, providing the compound Ce₆-APTS-1. After 3-4 hrs, the Ce₆-APTS-1 is reacted with 1000 µL of TEOS or TMOS for 2-3 hrs in 10ml of 99% ethanolic medium, forming the precursor for silane coupled quasi-aggregated nanophotomedicine. The magnetic agents are doped within the nanomatrix during the hydrolysis and condensation of this precursor by the addition of 3 ml of water containing 0.01M iron chloride (FeCl3) followed by 800 µL NH₄O₄ under sonication for 10 minutes leading to the precipitation of nanoparticles of silicondioxide complexed with quasi-aggregated Ce₆ and doped with Fe³⁺, which remain embedded within the amorphous phase of the nanoparticulate matrix. The precipitated nanoparticles are separated from the ethanolic medium by centrifugation and are washed with distilled water before being re-dispersed into PBS solution.

Magnetic studies carried out using vibrating sample magnetometer revealed the paramagnetic property (Fig.7) of the Gd³⁺ doped nanophotomedicine compared to the diamagnetic response of free Ce₆. Further, the applicability of this system for magnetic resonance imaging was demonstrated by imaging a collection of ∼ 80.000 cancer cells treated with the doped nanophotomedicine in a 24 well plate using a clinical MRI unit at 1.5T. Fig. 8 shows the T1 weighed contrast imaging of the cells treated with the doped nanophotomedicine of different concentration together with control (untreated cells) and free Ce₆ treated cells. It can be seen that the contrast increases with the concentration of nanophotomedicines, confirming that the doped nanophotomedicines described in the present invention can be used for MRI based diagnosis together with photodynamic therapy. This has significance in pre-therapeutic planning, understanding the pharmacokinetics of the administrated drug using completely non-invasive technique and post-treatment efficacy analysis.

### Example 11

In this example, the delivery of peptide-conjugated nanophotomedicine to cancer cells and photodynamic therapy by sensitizing the conjugated nanophotomedicine using a red laser (emission 652nm) is described.

Leukemia cells K562 were seeded at 800.000 cells / well in a 96 well microtiter plate and treated with free Ce₆ (1 µM), 0.05mg/ml nanophotomedicine (NPM-3) providing an equal concentration of sensitizer as the free photosensitizer, and 0.05mg/ml bare silica nanoparticles as control. Cells were incubated at 37°C for 3 hrs under 5% CO₂. Subsequently, unattached free sensitizer, nanophotomedicine and silica nanoparticles were removed from the well plate and washed 2 times with fresh medium.

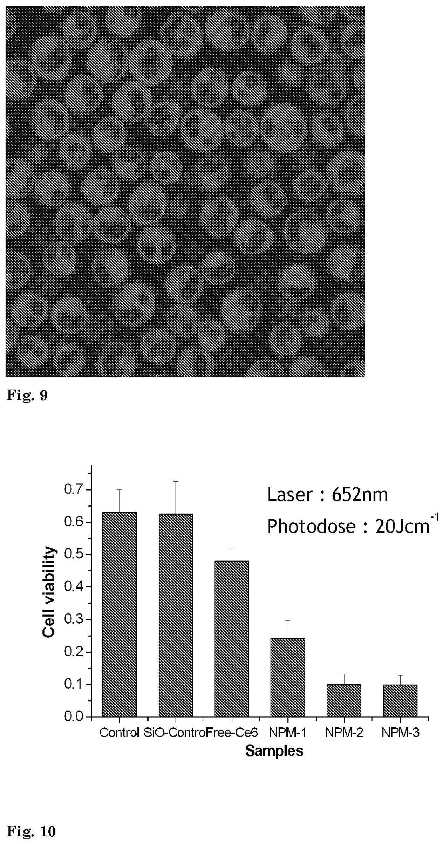

A test sample was used to study the cellular uptake of the peptide conjugated nanophotomedicine. Fig.9 shows significant subcellular uptake of the conjugated nanophotomedicine by cancer cells, confirming the successful drug delivery.

Subsequently, PDT was carried out using a solid state laser emitting 652 nm coherent light coupled through a fiber optic irradiator, which delivers uniform laser power over all wells of the 96 well plate. A total light dose of 20 J cm⁻² was applied as measured using a laser power meter. An amount of 5mW of laser power was delivered over a period of 4000 sec to achieve the total dose of 20 J cm⁻².

Following PDT, cells were further incubated for 72 days to evaluate the cell viability and proliferative capacity of the treated cells using a standard assay (Roche Cell Proliferation Reagent WST-1, Roche Diagnostics GmbH, Mannheim, Germany) which uses optical absorption (optical density) of formazan crystals (480nm) formed in the mitochondria of the viable cells due to metabolic activity of these cells. This test therefore provides direct information about cell death/viability due to the PDT treatment.

Fig. 10 shows the result of the WST assay. These data clearly suggest that, compared to free photosensitizer, all three nanophotomedicine constructs shows higher therapeutic effect (killing of cancer cells). This confirms the advantageous property of the said constructs in photodynamic therapy.

## Claims

1. A method for the production of a photosensitizer-containing nanoparticle suitable for use in molecular imaging assisted targeted photodynamic therapy comprising:
a) providing a nanoparticle precursor molecule;
b) coupling a photosensitizer to said nanoparticle precursor molecule to provide a photosensitizer-conjugated nanoparticle precursor,
c) forming a nanoparticle from said photosensitizer-nanoparticle precursor mixture resulting from step b) by solution-precipitation or molecular self assembly.

2. A method according to claim 1, wherein the method further comprises a step comprising adding a magnetic and/or optical contrast agent to the photosensitizer-nanoparticle precursor conjugate to provide a photosensitizer-nanoparticle precursor mixture, wherein the step of adding magnetic and/or optical contrast agent is after the step of coupling the photosensitizer (b) and before the step of forming a nanoparticle (c).

3. A method according to claim 1 or 2, wherein said nanoparticles is formed from a material selected from the group consisting of metal sulphate, metal phosphate, metal oxide, chitosan, carboxymethyl chitosan (CMC), polyvinyl alcohol (PVA), polystyrene (PS) polyvinylpyrrolidone (PVP), polylactic acid (PLA), polyethylenimine (PEI), poly(lactic-co-glycolic acid) (PLGA), polycaprolactone (PCL), polyethylene glycol (PEG), and combinations thereof.

4. A method according to claim 3, wherein said metal oxide is silica, wherein said precursor molecule is an orthosilicate and wherein said nanoparticle is formed by process of hydrolysis and condensation of orthosilicate precursors under conditions of basic pH and under sonication to form colloidal silica nanoparticles.

5. Method according to any one of claims 1 -3, wherein said photosensitizer is selected from chlorin e₆ (Ce₆), meso-tetra(3-hydroxyphenyl)chlorin (m-THPC), benzoporphyrin derivative monoacid ring A (BPD or verteporfin), photofrin, temoporfin (Foscan®), Rose bengal, metal phthalocyanine and combinations thereof.

6. A photosensitizer-containing nanoparticle obtainable by a method according to any one of claim 1-5.

7. A photosensitizer-containing nanoparticle, comprising a photosensitizer covalently bonded throughout at least a part of said nanoparticle to the nanoparticle matrix material and incorporated therein as a mixture of monomeric and aggregated molecules, wherein the ratio of Q band absorption to Soret band absorption of said nanoparticles has a value of at least 0.3.

8. A nanoparticle according to claim 6 or 7, wherein said nanoparticle is formed from a material selected from the group consisting of metal sulphate, metal phosphate, metal oxide, carboxymethyl chitosan (CMC), polyvinyl alcohol (PVA), polystyrene (PS) polyvinylpyrrolidone (PVP), polylactic acid (PLA), polyethylenimine (PEI), poly(lactic-co-glycolic acid) (PLGA), polycaprolactone (PCL), polyethylene glycol (PEG),and combinations thereof.

9. A nanoparticle according to claim 8, wherein said metal oxide is silica.

10. A nanoparticle according to any one of claims 6-9, wherein said photosensitizer is selected from chlorin e₆ (Ce₆), meso-tetra(3-hydroxyphenyl)chlorin (m-THPC), benzoporphyrin derivative monoacid ring A (BPD or verteporfin), photofrin, temoporfin (Foscan®), Rose bengal, metal phthalocyanine and combinations thereof.

11. A nanoparticle according to any one of claims 6-10, wherein said nanoparticle is doped with an optical contrast agent and/or a magnetic contrast functionality, preferably wherein the optical contrast agent is luminescent quantum dots of ZnS doped with Mn²⁺, Cu⁺-Al³⁺ or Cu⁺-halogen or combinations thereof, or preferably wherein the magnetic contrast functionality is provided by doping the nanophotomedicine with Gd³⁺, Fe³⁺ or Mn²⁺.

12. A nanoparticle according to any one of claims 6-11, wherein said nanoparticle comprises a cancer-targeting ligand connected to the outermost surface through covalent linkage.

13. A nanoparticle according to claim 12, wherein the cancer-targeting ligand is octreotide or octreotate or their carboxylate derivatives such as DTPA-Tyr3-octreotide, DOTA- Tyr3-octreotide, DTPA -Tyr3-octreotate or DOTA -Tyr3- octreotate that targets the somatostatin receptor type 2.

14. An injectable composition or composition for oral administration comprising the nanoparticles according to any one of claims 6-13 together with a pharmaceutically acceptable carrier.

15. Nanoparticle according to any one of claims 6-13 for use in a method of killing cancer cells by PDT treatment, comprising contacting said cancer cells with said nanoparticle and irradiating said nanoparticles with a therapeutically effective amount of light so as to evoke singlet oxygen emission from said nanoparticles.

16. Nanoparticle according to any one of claims 6-13 for use in a method of killing cancer cells by image assisted PDT treatment, comprising contacting said cancer cells with said nanoparticle and irradiating said nanoparticles with a therapeutically effective amount of light so as to evoke singlet oxygen emission from said nanoparticles, wherein the nanoparticle is doped with an optical contrast agent and/or a magnetic contrast agent and wherein the direction of said irradiation is guided by imaging techniques that use the optical or magnetic contrast agent as markers to indicate the location, size and spread of the cancer cells.

## Patentansprüche

1. Verfahren zur Herstellung eines einen Fotosensibilisator enthaltenden Nanopartikels, geeignet zur Verwendung bei durch molekulare Bildgebung unterstützter, zielgerichteter photodynamischer Behandlung, umfassend:
a) Bereitstellen eines Nanopartikel-Vorläufermoleküls;
b) Koppeln eines Fotosensibilisators an das Nanopartikel-Vorläufermolekül um einen Fotosensibilisator-konjugierten Nanopartikel-Vorläufer bereitzustellen,
c) Bilden eines Nanopartikels von dem aus Schritt b) resultierenden Fotosensibilisator-Nanopartikel-Vorläufergemisch durch Lösungsausfällung oder molekulare Selbstanordnung.

2. Verfahren nach Anspruch 1, wobei das Verfahren ferner einen Schritt umfasst, umfassend Hinzufügen eines magnetischen und/oder optischen Kontrastmittels zu dem Fotosensibilisator-Nanopartikel-Vorläuferkonjugat, um ein Fotosensibilisator-Nanopartikel-Vorläufergemisch bereitzustellen, wobei der Schritt des Hinzufügens des magnetischen und/oder optischen Kontrastmittels nach dem Schritt des Koppelns des Fotosensibilisators (b) und vor dem Schritt des Bildens eines Nanopartikels (c) erfolgt.

3. Verfahren nach Anspruch 1 oder 2, wobei die Nanopartikel aus einem Material gebildet werden, ausgewählt aus der Gruppe bestehend aus Metallsulfat, Metallphosphat, Metalloxid, Chitosan, Carboxymethylchitosan (CMC), Polyvinylalkohol (PVA), Polystyrol (PS), Polyvinylpyrrolidon (PVP), Polylactidsäure (PLA), Polyethylenimin (PEI), Poly(Lactid-co-Glykolsäure) (PLGA), Polycaprolacton (PCL), Polyethylenglykol (PEG) und Kombinationen davon.

4. Verfahren nach Anspruch 3, wobei das Metalloxid Siliziumdioxid ist, wobei das Vorläufermolekül ein Orthosilikat ist und wobei das Nanopartikel gebildet wird durch den Prozess von Hydrolyse und Kondensation von Orthosilikat-Vorläufern unter Bedingungen von basischem pH und bei Ultraschallbehandlung, um kolloidale Siliziumdioxid-Nanopartikel zu bilden.

5. Verfahren nach einem der Ansprüche 1-3, wobei der Fotosensibilisator ausgewählt ist aus Chlorin e₆ (Ce₆), meso-Tetra(3-hydroxyphenyl)chlorin (m-THPC), Benzoporphyrin-Derivat-Monosäure-Ring-A (BPD oder Verteporfin), Photofrin, Temoporfin (Foscan®), Bengalrosa, Metallphthalocyanin und Kombinationen davon.

6. Fotosensibilisator enthaltendes Nanopartikel, erhältlich durch ein Verfahren nach einem der Ansprüche 1-5.

7. Fotosensibilisator enthaltendes Nanopartikel, umfassend einen Fotosensibilisator, kovalent durch mindestens einen Teil des Nanopartikels an das Nanopartikel-Matrixmaterial gebunden und darin eingebunden als ein Gemisch von monomeren und aggregierten Molekülen, wobei das Verhältnis von Absorption der Q-Bande zu Absorption der Soret-Bande der Nanopartikel einen Wert von mindestens 0,3 hat.

8. Nanopartikel nach Anspruch 6 oder 7, wobei das Nanopartikel aus einem Material gebildet wird, ausgewählt aus der Gruppe, bestehend aus Metallsulfat, Metallphosphat, Metalloxid, Carboxymethylchitosan (CMC), Polyvinylalkohol (PVA), Polystyrol (PS), Polyvinylpyrrolidon (PVP), Polylactidsäure (PLA), Polyethylenimin (PEI), Poly(Lactid-co-Glykolsäure) (PLGA), Polycaprolacton (PCL), Polyethylenglykol (PEG) und Kombinationen davon.

9. Nanopartikel nach Anspruch 8, wobei das Metalloxid Siliziumdioxid ist.

10. Nanopartikel nach einem der Ansprüche 6-9, wobei der Fotosensibilisator ausgewählt ist aus Chlorin e₆ (Ce₆), meso-Tetra(3-hydroxyphenyl)chlorin (m-THPC), Benzoporphyrin-Derivat-Monosäure-Ring-A (BPD oder Verteporfin), Photofrin, Temoporfin (Foscan®), Bengalrosa, Metallphthalocyanin und Kombinationen davon.

11. Nanopartikel nach einem der Ansprüche 6-10, wobei das Nanopartikel mit einem optischen Kontrastmittel und/oder einer magnetischen Kontrastfunktionalität dotiert ist, bevorzugt wobei es sich bei dem optischen Kontrastmittel um lumineszente Quantenpunkte von ZnS handelt, dotiert mit Mn²⁺, Cu⁺-Al³⁺ oder Cu⁺-Halogen oder Kombinationen davon, oder bevorzugt wobei die magnetische Kontrastfunktionalität durch Dotieren der Nanofotomedizin mit Gd³⁺, Fe³⁺ oder Mn²⁺ bereitgestellt wird.

12. Nanopartikel nach einem der Ansprüche 6-11, wobei das Nanopartikel einen auf Krebs-zielgerichteten Liganden umfasst, der mit der äußersten Oberfläche durch kovalente Bindung verbunden ist.

13. Nanopartikel nach Anspruch 12, wobei der auf Krebs-zielgerichtete Ligand Octreotid oder Octreotat ist, oder deren Carboxylat-Derivate wie etwa DTPA-Tyr3-Octreotid, DOTA-Tyr3-Octreotid, DTPA-Tyr3-Octreotat oder DOTA-Tyr3-Octreotat, der auf den Somatostatin Typ-2 Rezeptor abzielt.

14. Injizierbare Zusammensetzung oder Zusammensetzung zur oralen Verabreichung, umfassend die Nanopartikel nach einem der Ansprüche 6-13, zusammen mit einem pharmazeutisch unbedenklichen Träger.

15. Nanopartikel nach einem der Ansprüche 6-13 zur Verwendung in einem Verfahren zum Abtöten von Krebszellen durch PDT-Behandlung, umfassend Kontaktieren der Krebszellen mit dem Nanopartikel und Bestrahlen der Nanopartikel mit einer therapeutisch wirksamen Lichtmenge, um Singulett-Sauerstoffemission von den Nanopartikeln hervorzurufen.

16. Nanopartikel nach einem der Ansprüche 6-13 zur Verwendung in einem Verfahren zum Abtöten von Krebszellen durch bildunterstützte PDT-Behandlung, umfassend Kontaktieren der Krebszellen mit dem Nanopartikel und Bestrahlen der Nanopartikel mit einer therapeutisch wirksamen Lichtmenge, um Singulett-Sauerstoffemission von den Nanopartikeln hervorzurufen, wobei das Nanopartikel mit einem optischen Kontrastmittel und/oder einem magnetischen Kontrastmittel dotiert ist und wobei die Richtung der Bestrahlung durch Bildgebungstechniken geleitet wird, die das optische oder magnetische Kontrastmittel als Marker zum Anzeigen der Stelle, Größe und Ausbreitung der Krebszellen verwenden.

## Revendications

1. Procédé de production d'une nanoparticule comportant un photo-sensibilisateur, appropriée pour être utilisée en thérapie photodynamique ciblée assistée par imagerie moléculaire, comprenant les étapes suivantes :
a) prendre une molécule précurseur de nanoparticule ;
b) coupler un photo-sensibilisateur à ladite molécule précurseur de nanoparticule, pour en faire un précurseur de nanoparticule conjugué à un photo-sensibilisateur,
c) et former une nanoparticule à partir dudit mélange de précurseur de nanoparticule et de photo-sensibilisateur issu de l'étape (b), par dissolution et précipitation ou par auto-assemblage moléculaire.

2. Procédé conforme à la revendication 1, lequel procédé comporte en outre une étape comprenant le fait d'ajouter un agent de contraste optique et/ou magnétique au conjugué de photo-sensibilisateur et de précurseur de nanoparticule pour obtenir un mélange de précurseur de nanoparticule et de photo-sensibilisateur, laquelle étape d'addition d'un agent de contraste optique et/ou magnétique prend place après l'étape (b) de couplage du photo-sensibilisateur et avant l'étape (c) de formation d'une nanoparticule.

3. Procédé conforme à la revendication 1 ou 2, dans lequel ladite nanoparticule est formée à partir d'un matériau choisi dans l'ensemble constitué par les suivants : sulfate de métal, phosphate de métal, oxyde de métal, chitosane, carboxyméthyl-chitosane (CMC), poly(alcool de vinyle) (PVA), polystyrène (PS), poly(vinyl-pyrrolidone) (PVP), poly(acide lactique) (PLA), poly(éthylène-imine) (PEI), poly(acide lactique-co-glycolique) (PLGA), polycaprolactone (PCL) et poly(éthylène-glycol) (PEG), ainsi que leurs combinaisons.

4. Procédé conforme à la revendication 3, dans lequel ledit oxyde de métal est de la silice, ladite molécule précurseur est un orthosilicate et l'on forme ladite nanoparticule par un procédé d'hydrolyse et condensation des précurseurs ortho-silicates, à pH basique et avec sonication, pour former des nanoparticules de silice colloïdale.

5. Procédé conforme à l'une des revendications 1 à 3, dans lequel ledit photo-sensibilisateur est choisi parmi les suivants : chlorine e₆ (Ce₆), méso-tétra(3-hydroxy-phényl)chlorine (m-THPC), dérivé monoacide (cycle A) de benzoporphyrine (BPD ou vertéporfine), Photofrin, témoporfine (Foscan^{®}), rose bengale et phtalocyanine de métal, et leurs combinaisons.

6. Nanoparticule comportant un photo-sensibilisateur, accessible par un procédé conforme à l'une des revendications 1 à 5.

7. Nanoparticule comportant un photo-sensibilisateur, comprenant un photo-sensibilisateur lié par liaison covalente, dans au moins une partie de ladite nanoparticule, au matériau de matrice de la nanoparticule et incorporé là-dedans sous forme d'un mélange de molécules monomères et de molécules agrégées, et dans laquelle nanoparticule le rapport de l'absorption à la bande Q à l'absorption à la bande de Soret vaut au moins 0,3.

8. Nanoparticule conforme à la revendication 6 ou 7, laquelle nanoparticule est formée à partir d'un matériau choisi dans l'ensemble formé par les suivants : sulfate de métal, phosphate de métal, oxyde de métal, chitosane, carboxyméthyl-chitosane (CMC), poly(alcool de vinyle) (PVA), polystyrène (PS), poly(vinyl-pyrrolidone) (PVP), poly(acide lactique) (PLA), poly(éthylène-imine) (PEI), poly(acide lactique-co-glycolique) (PLGA), polycaprolactone (PCL) et poly(éthylène-glycol) (PEG), ainsi que leurs combinaisons.

9. Nanoparticule conforme à la revendication 8, dans laquelle ledit oxyde de métal est de la silice.

10. Nanoparticule conforme à l'une des revendications 6 à 9, dans laquelle ledit photo-sensibilisateur est choisi parmi les suivants :
chlorine e₆ (Ce₆), méso-tétra(3-hydroxy-phényl)chlorine (m-THPC), dérivé monoacide (cycle A) de benzoporphyrine (BPD ou vertéporfine), Photofrin, témoporfine (Foscan^{®}), rose bengale et phtalocyanine de métal, et leurs combinaisons.

11. Nanoparticule conforme à l'une des revendications 6 à 10, laquelle nanoparticule est dopée avec un agent de contraste optique et/ou une fonction de contraste magnétique, étant entendu que, de préférence, l'agent de contraste optique est constitué par des points quantiques luminescents de sulfure de zinc (ZnS) dopé avec des ions Mn²⁺, Cu⁺-Al³⁺ ou Cu⁺-halogène ou une combinaison de ceux-ci, ou que, de préférence, la fonction de contraste magnétique est assurée par dopage du nano-photo-médicament avec des ions Gd³⁺, Fe³⁺ ou Mn²⁺.

12. Nanoparticule conforme à l'une des revendications 6 à 11, laquelle nanoparticule comprend un ligand pour ciblage de cancer, attaché à sa surface externe par un raccord covalent.

13. Nanoparticule conforme à la revendication 12, dans laquelle le ligand pour ciblage de cancer est l'octréotide ou l'octréotate, ou l'un de leurs dérivés carboxylates, tels les DTPA-Tyr3-octréotide, DOTA-Tyr3-octréotide, DTPA-Tyr3-octréotate et DOTA-Tyr3-octréotate, qui a pour cible les récepteurs de type 2 de la somatostatine.

14. Composition injectable ou composition pour administration par voie orale, comprenant des nanoparticules conformes à l'une des revendications 6 à 13, conjointement avec un véhicule pharmacologiquement admissible.

15. Nanoparticules conformes à l'une des revendications 6 à 13 pour utilisation dans un procédé visant à tuer des cellules cancéreuses par thérapie photodynamique, comportant le fait de mettre lesdites cellules cancéreuses en contact avec lesdites nanoparticules et le fait d'irradier ces nanoparticules avec de la lumière utilisée en une quantité à effet thérapeutique, de manière à provoquer l'émission d'oxygène singulet par lesdites nanoparticules.

16. Nanoparticules conformes à l'une des revendications 6 à 13 pour utilisation dans un procédé visant à tuer des cellules cancéreuses par thérapie photodynamique assistée par imagerie, comportant le fait de mettre lesdites cellules cancéreuses en contact avec lesdites nanoparticules et le fait d'irradier ces nanoparticules avec de la lumière utilisée en une quantité à effet thérapeutique, de manière à provoquer l'émission d'oxygène singulet par lesdites nanoparticules, dans lequel procédé les nanoparticules sont dopées avec un agent de contraste optique et/ou un agent de contraste magnétique, et l'on détermine la direction de ladite irradiation au moyen de techniques d'imagerie, en utilisant l'agent de contraste optique ou magnétique comme marqueur pour indiquer l'emplacement ou la taille des cellules cancéreuses ou l'étendue de leur diffusion.
